(19) Europäisches Patentamt

European Patent Office

Office européen des brevets

(11) **EP 1 595 550 A1**

(12) **EUROPEAN PATENT APPLICATION**

(43) Date of publication:
**16.11.2005 Bulletin 2005/46**

(51) Int Cl.⁷: **A61K 47/48**, A61K 51/04, A61K 49/00

(21) Application number: **04011139.5**

(22) Date of filing: **11.05.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK** | (72) Inventor: **Neirinckx, Rudi D., Dr.**<br>**8008 Zürich (CH)** |
| | Remarks:<br>Amended claims in accordance with Rule 86 (2) EPC. |
| (71) Applicant: **Neirinckx, Rudi D., Dr.**<br>**8008 Zürich (CH)** | |

(54) **Selective retention of anti-cancer therapeutics in tumour cells through intra-cellular reaction with heat shock proteins**

(57)    The invention presented concerns a selective intracellular retention of a desired molecule through the conversion of a freely-diffusible molecule -metal complex or organic molecule-into a retained species through the use of intracellular reactions , the rate of which depends on the level of concentration of a reactant which is especially elevated in tumour cells and, more specifically, in tumour cells which have a high resistance against chemotherapeutic agents. To use this invention one needs to identify an anti-tumour compound which - although degraded by the intracellular reactant - retains its cytotoxic action in the cell.

One of the mechanisms which could be used under this invention is the reaction of GSH, catalysed by the Glutathione S- Transferases (GST) family of enzymes, with a variety of target compounds. This is possible because the presence of high GSH concentrations is a feature of many resistant cancer clones , and this opens the possibility to selectively destroy these high-GST/ GSH cells

A major additional advantage when using this mechanism is the fact that screening for molecules with the suitable pharmaceutical properties is straightforward because one can use a simple in-vitro test.

## Description

### Summary

The invention presented here concerns a selective intracellular retention of a desired molecule through the conversion of a freely-diffusible molecule -metal complex or organic molecule-into a retained species through the use of intracellular reactions, the rate of which depends on the level of concentration of a reactant which is especially elevated in tumour cells and, more specifically, in tumour cells which have a high resistance against chemotherapeutic agents.

### Description

Drugs can have their efficacy/safety profile - or therapeutic index (T.I.) - improved if they could be retained more selectively in the target cell. The T.I. of anti-cancer drugs would be much higher if they could be enriched in the tumour tissue.

Such enrichment can in principle be obtained through a variety of procedures, e.g.

1. the active transport into the cell
2. the "metabolic trapping" of a relatively-freely diffusible compound
3. attachment of the molecule to a receptor bound molecule such as an antibody, ....

And such procedures have been attempted in oncology with varying degrees of success.

### Proposal

The invention presented here concerns a selective intracellular retention of a desired molecule through the conversion of a freely-diffusible molecule -metal complex or organic molecule-into a retained species through the use of intracellular reactions, the rate of which depends on the level of concentration of a reactant which is especially elevated in tumour cells and, more specifically, in tumour cells which have a high resistance against chemotherapeutic agents.

Chemical compounds of this invention which can be selectively retained in certain cell types should have the following features:

1. The molecule must be able to cross the cell membrane
2. The molecule must be the substrate for an intracellular metabolic reaction which converts the substrate into retained reaction products.
3. The conversion should be driven by a reaction which is fast and has different reaction rates (k) in different cell types in order to allow a differentiation between these different cell types.
4. The reactant driving the reaction should be present in higher concentrations in the target tissue than in the surrounding tissue

Depending on the value of the reaction rate and the concentration of the intracellular reactant - the relative retention rate will be either a reflection of the reactant concentration or of the relative delivery rate to the site(e.g. regional blood flow). In case the reaction is slow (low k value) the relative concentrations of the retained ("trapped") form of the molecule will be proportional to the concentration of the intracellular reactant. If the reaction is fast when compared to the rate of return of the non-metabolised molecule back to the extra cellular space more compound will remain intracellular, but its distribution will reflect more the local blood flow rather than the concentration of reactant There will therefore be a trade-off between quantitative uptake and selectivity in retention . See below.

This principle can be used to improve the therapeutic ratio for cancer drugs, for example for cyto-toxic molecules (see further) or for nucleic acid products by making their retention more selective in certain target cell populations if the "Retention reaction" is faster in the target tissue than in the surrounding tissue. As explained above, this requires that the responsible reactant be present in higher concentration in the target tissue.

Specifically, this invention aims to use a mechanism which lies at the basis of resistance of certain tumour cells against chemotherapy . Such resistance is often caused by the enhanced metabolism and excretion of the drug through the action of " Heat Shock Proteins" , e.g. the GST-mediated reaction of the drug with GSH: Some tumour cells can become resistant to chemotherapeutic agents if they contain high levels of these "Heat shock proteins", such as

GST . This invention can improve the therapeutic action of certain cyto-toxic compounds through the use of the GST/GSH driven metabolic process which enriches the cyto-toxic agent, as long as the cyto-toxic properties themselves are not affected by the metabolic reaction itself.

This may represent a major advance in the struggle against therapy-resistant tumours.

**Mathematical explanation of the trapping principle**

If a certain compound, X, can be made to react with an intracellular component such as GST/GSH, then the relative distribution in different tissues can be calculated as follows:

$$D = e^{-k_5.T}.C.V.k_3/(k_3+k_2) \qquad \text{(Formula 1)}$$

Where

- $k_2$ is the rate of return of the non-metabolised compound X to the blood,
- $k_3$ is the reaction rate describing the intracellular metabolism of X
- and $k_5$ is the rate of the metabolic reaction of the compound X in the blood .
- T is the time the compound X resides in the blood between administration and uptake in the target cancer cell or in the healthy tissue,
- C is the fractional cardiac output to the tumour or the healthy tissue and
- V is the fraction of non-metabolised drug X remaining in the blood at the time it reaches the organ or tumour under consideration.

The metabolic reaction rates, which are assumed to be primarily dependent on the GSH-concentration, are proportional to the concentration of GSH , i.e.

$$k_3 = k_{GSH} * (GSH)_{tissue}$$

and

$$k_5 = k_{GSH} * (GSH)_{blood}$$

where $(GSH)_{tissue}$ and $(GSH)_{blood}$ are the concentrations of GSH in tissue and blood respectively and $k_{GSH}$ is the reaction constant between the molecule of interest and GSH.

**$k_3$ is small relative to $k_2$**

If $k_3$ is relatively small the calculation of D can be simplified to

$$D = e^{-k_5.T}.C.V.k_3/k_2$$

Or

$$D = e^{-k_5.T}.C.V. k_{GSH} * (GSH)_{tissue}/k_2$$

and thus D becomes proportional to the concentration of GSH in the tissue. The retention in each tissue (tumour and normal surrounding tissue) can be calculated for a product ,X, which is subject to metabolism driven by GSH ,and another product ,Y, which is not subject to such metabolism.

Under the assumption that for the metabolised compound ,X , $k_3$ is relatively small:

$$D_{X,C} = e^{-k_5.T}.C_C.V_{X,C}.k_{3,C}/k_{2,C}$$

$$D_{X,N} = e^{-k_5.T}.C_N.V_{X,N}.k_{3,N}/k_{2,N}$$

$$D_{Y,C} = C_C \cdot V_{Y,C}$$

$$D_{Y,N} = C_N \cdot V_{Y,N}$$

Where $D_{X,C}$, $D_{X,N}$, $D_{Y,C}$ and $D_{Y,N}$ are the calculated distributions of compound X in the cancer tissue, the calculated distributions of compound X in the normal tissue ,the calculated distributions of compound Y in the cancer tissue and the calculated distributions of compound Y in the normal tissue respectively and

Where $C_C$ and $C_N$ are the cardiac outputs to the cancer tissue and the normal cells respectively and

Where $V_{X,C}$, $V_{X,N}$, $V_{Y,C}$ and $V_{Y,N}$ are the fractions of drug X remaining in the blood at the time it reaches the cancer cells (C) the normal tissue (N) and the fractions of drug Y remaining in the blood at the time it reaches the cancer cells (C) the normal tissue (N)

Because $k_5$ and V are identical for the same compound and $k_2$ is identical for all tissues the ratio of $D_{X,C}$ and $D_{X,N}$ describes the relative enrichment of the tumour drug X in the cancer cells (C) versus the normal surrounding tissue (N) and is

$$R_X = D_{X,C} / D_{X,N} = (C_C / C_N) \cdot (k_{3,C} / k_{3,N}) \qquad \text{(Formula 2)}$$

The ratio of $D_{Y,C}$ and $D_{Y,N}$ describes the relative enrichment of the tumour drug Y in the cancer cells (C) versus the normal surrounding tissue (N) and is

$$R_Y = D_{Y,C} / D_{Y,N} = (C_C / C_N) \cdot (V_{Y,C} / V_{Y,N})$$

The relative improvement of the metabolised compound, X , over the non-metabolised compound, Y , in terms of enrichment in the cancer cells over the normal tissue is:

$$R_X / R_Y = (k_{3,C} / k_{3,N}) / (V_{Y,C} / V_{Y,N})$$

For cancer cells and their surrounding tissue the value of V is very similar and therefore

$$R_X / R_Y = k_{3,C} / k_{3,N} = (k_{GSH} * (GSH)_{cancer}) / (k_{GSH} * (GSH)_{normal\ tissue})$$

i.e. the relative improvement in retention in the cancer tissue of the cancer drug X over the drug Y is proportional to the ratio of the GSH levels in the cancer tissue versus that in the normal tissue.
This demonstrates that this principle will lead to better Therapeutic Index. for the drug X.

**$k_3$ is large relative to $k_2$**

It will be clear from the basic formula (1) and (2) that when $k_3$ is high in comparison with $k_2$ the ratio of the distribution in cancerous and normal tissue distribution ,$R_X$, will become

$$R_X = D_{X,C} / D_{X,N} = (C_C / C_N) \cdot \{(k_{3,C} / (k_{3,C} + k_{2,C})\} \cdot \{(k_{3,N} + k_{2,N}) / k_{3,N} \}$$

Where Rx will approach $C_C / C_N$ more and more as the values of $k_2$ become smaller and smaller when compared to the values of $k_3$.

Although in this case the absolute value of $D_{X,C}$ will increase ,the ratio ($R_X$) will decrease and there will be a trade-off between quantitative effect (damage to the tumour cells), determined by $D_{X,C}$ and the qualitative effect (Therapeutic Index) determined by $R_X$.
These will need to be balanced ,taking into account the fate of the cyto-toxic drug (pharmacokinetics, pharmacodynamics) which has not been taken up by the tumour.

**Difference with the "metabolic retention" of certain anti tumour compounds:**

So-called "metabolic retention" has been previously described to be important for the retention of certain antitumour antibiotics- such as Doxorubicin- inside cells. These retention mechanisms and reactions are different from what is described here and do not form part of this invention because they make a minor change to the cytotoxic agent . For example,for Doxorubicin the reaction is a simple reduction of a carbonyl function which increases the hydrophilicity of the original molecule and which therefore make it less likely to permeate the cell membrane, leaving it vulnerable to the same cellular resistance mechanisms as the original molecule: The slightly-modified structure will remain subject to the action of the "Heat Shock Proteins" and it will be eliminated.

Furthermore, this reduction reaction occurs in most cells and does not lend selectivity for any retention in the targeted tumour cells, i.e. in this case the values of $k_{3,C}$ and $k_{3,N}$ would be identical and

$$R_X / R_Y = 1$$

The product would be retained similarly in a large number of different cell types.

A confirmation of this problem is that attempts have been made recently to improve the ratios tumour/non-tumour through the use of internalised monoclonal antibodies which have doxorubicin attached to them .

**Preferred metabolic reaction: Reaction with the "Heat shock proteins " such as GST/GSH**

As stated earlier, one of the mechanisms which could be used under this invention is the reaction of GSH , catalysed by the Glutathione S- Transferases (GST) family of enzymes, with a variety of target compounds: There is ample evidence suggesting that the level of expression of GST is a crucial factor in determining the level of resistance which cells can develop to a broad spectrum of toxic chemicals. GST belongs to the family of "chaperone proteins" called "Heat shock proteins" which serve to promote survival under adverse conditions They confer resistance to chemotherapy and radiation-induced apoptosis to tumour cells. Some of these GST-transferases have specific roles in the binding and transport of ligands such as bile acids, haematin and fatty acids. The GSTP1 member of the GST family acts on DNA and oxidized fats, the GSTM1 member acts on cyclical hydrocarbons, ... The GST family members also act on cytotoxic anti-cancer agents and seem to be responsible for the development of resistant cancer cell lines, when the GST concentration is high. The glutathione S-transferases catalyze the addition of the tripeptide glutathione (GSH) to endogenous and xenobiotic substrates which have electrophilic functional groups. They play an important role in the detoxification and metabolism of these compounds .

Overproduction of GST is one of the recognized pathways which tumour cells use to produce resistance to anti-cancer drugs. In particular, all alkylating agents - a very important class of anti-tumour drugs- may be rendered ineffective by the action of GST .The Glutathione S-transferases (GSTs) can employ either glutathione conjugation, glutathione peroxidase activity or passive/sacrificial binding. The glutathione adducts produced are subsequently enzymatically degraded to mercapturates and excreted.

The presence of high GSH concentrations is a feature of many resistant cancer clones, and this opens the possibility to selectively destroy these high-GST cells: As long as the reaction product of an anti-tumour product with the GST/GSH system is not excreted and remains able to destroy biological material it would lead to a selective killing of the "resistant cells". This is the case with complexes of radioactive isotopes: If they can be transported into the cells and concentrate proportionately to the concentration of GST/GSH their action would be most pronounced on those cells which feature the highest concentrations of GST/GSH, i.e. the resistant tumour clones.

It therefore becomes possible to design anti-tumour compounds which- through the reaction with GSH - form reaction products which are not likely to be excreted by the cancer cell. In this way the defence mechanism of the tumour is turned upon itself . If the anticancer compounds can be designed to contain lipophilic groups which facilitate the transition through the membrane , but which-through the reaction with GSH - would decompose and becomes less likely to cross the membrane it will have more time to act on the tumour.

Crucial here is the need to concentrate these compounds proportionally to the GSH concentration. It can be easily shown mathematically that this requires that the metabolism of the compound in question happens relatively slow in comparison with the blood flow which brings it to the tissue. The difference in concentration of GST/GSH between normal tissue and tumour tissue will then determine the difference in radiation damage suffered by each tissue.

**Example:**

An example of this mechanism is the behaviour of certain complexes of propylene-amine oxime (PnAO), which react with glutathione (GSH) .This reaction has been used successfully to trap Tc-99m complexes of PnAO in brain tissue and other cellular systems (such as lymphocytes) high in concentration in the free-SH containing glutathione (GSH).

(See Ref 1)

This has successfully been used to image rCBF (regional cerebral blood flow). In this instance the reaction used was the metabolism of the Tc-99m complex into a radioactive hydrophilic forms which was retained intra-cellular. Tissues low in GSH left the complex unchanged and let it diffuse back out of the cell while the high -GSH tissues-such as brain-retained the more hydrophilic metabolic products which had been generated within the cell and which still contained the Tc-99m isotope which became trapped in the cell. In this case the metabolism of the Tc-99m compound was fast when compared with the local blood flow and therefore the concentration of the Tc-99m containing metabolite was proportional to the blood flow. Should the conversion of the radioactive compound have been slower, the distribution of the metabolic products would have reflected the GSH-concentration rather than the blood flow.

Thus, to use this invention one needs to identify an anti-tumour compound which - although degraded by GSH - retains its cytotoxic action in the cell.

Example of these are compounds containing radioactive isotopes: These do not depend on chemical structure for their cytotoxic capabilities and therefore the "carrier" molecule which transports the isotope into the cell can be destroyed without affecting the efficacy of the isotope as long as the latter remains confined within the cell. Alpha, beta or gamma emitting radioisotopes can be used for this purpose and the relative enrichment in the tumour cells could be accomplished as long as the non-metabolised compound is able to exit from the cell. This will ensure that normal tissue, low in GSH, will receive a much lower radiation dose than the high-GSH tumour tissue. As one of the necessary conditions for the complex is that it be able to enter the cells is , it follows that the unchanged product will also be able to leave the normal tissue.

A major additional advantage when using this mechanism is the fact that screening for molecules with the suitable pharmaceutical properties is straightforward because one can use a simple in-vitro test which accurately predicts the relative retention in different tissues depending on their content of GST/GSH . This allows a fast optimisation of the ability of molecular structures capable of modulating membrane permeability and entrapment within the target cells.

## REFERENCES:

1. Rudi D. Neirinckx et al, J. of Cerebral Blood Flow and Metabolism 8: S4- S12,1988.
2. Douglas Hagrman, Jerry Goodisman, and Abdul-Kader Souid ;JPET 308:658-666, 2004)
3. Tadashi Arai et al; Cancer Detection and Prevention 2000; 24(3):252-257

## Claims

**1.** All cyto-toxic compounds which react with intracellular GSH and feature increased retention in high-GSH / high-GST intracellular environments.

**2.** All complexes or organic compounds containing therapeutic radioactive isotopes, according to Claim 1.

**3.** All preparations according to Claim 1, which are being used to selectively damage tumour cells.

**4.** All complexes and/or organic compounds according to Claim 2 where the isotope is I-131,I-125, Sr-89 or Y-90, but is not limited to those.

**5.** All compounds according to claim 1 which feature relatively modest reaction rates with GSH, so as to allow a differentiation tumour/normal cells based on their relative intracellular concentration of GSH.

**6.** All nucleic acid based products which can be retained intracellular through reaction with GST/GSH and can be used for anti-sense or gene therapy.

**Amended claims in accordance with Rule 86(2) EPC.**

**1.** All cyto-toxic compounds which react with intracellular GSH and feature increased retention in high-GSH / high-GST intracellular environments and maintain their cytotoxic character in the presence of high levels of GSH.

**2.** All compounds of Claim 1, where the cytotoxic properties, retained in the presence of GSH, is conferred by radioisotope radiation.

**3.** All compounds of Claim 1, where the cytotoxic properties, retained in the presence of GSH, is conferred by Platinum.

**4.** All compounds of Claim 3, where the ligands surrounding Platinum carry one or more groups, which can react with GSH and lead to a higher retention of Platinum.

**5.** All compounds of Claim 4, where the Pt-ion is linked to a diamino derivative of of one of the structures which are known to be degraded by GST (CNDB, 4-vinylpyridine, benzyl isothiocyanate, quinones, epoxides ,...)

**6.** All compounds of Claim 4, where the Pt-ion is linked to a ligand which contains an a β-unsaturated aldehyde, quinone or epoxide.

**7.** The product according to Claim 4, where a propenal group is added to the diaminocyclohexane group of oxaliplatin

**8.** All compounds and/or organic compounds according to Claim 2 where the isotope is 90Y, 99Mo, 111In, 123I, 186Re .18F, 32P, 81mKr, 89Sr, 103Pd, 117mSn, 127Xe, 125I, 131I, 153Sm, 47Sc, 62Zn, 64Cu, 67Cu, 68Ge, 153Gd, 168Ho, 177Lu, 188Re, 211At, 212Bi, 213Bi, 223Ra.

**9.** All compounds and/or organic compounds according to Claim 2 where the isotope is 131I, 90Y ,186Re, 32P, 89Sr , 103Pd, 117mSn, 153Sm .

**10.** All compounds according to claim 1, which feature relatively modest reaction rates with GSH, so as to allow a differentiation in their retention between cells with high-levels of GSH and cells with low-levels of. GSH.

**11.** All preparations according to Claim 1 through 10, which are being used to selectively damage and/or kill tumour cells.

**12.** A procedure for in-vitro screening of the compounds of Claim 1 through 10, based on the measurement of their reaction rate with GSH.

**European Patent Office**

## DECLARATION

which under Rule 45 of the European Patent Convention shall be considered, for the purposes of subsequent proceedings, as the European search report

**Application Number**

EP 04 01 1139

| The Search Division considers that the present application, does not comply with the provisions of the EPC to such an extent that it is not possible to carry out a meaningful search into the state of the art on the basis of all claims | CLASSIFICATION OF THE APPLICATION (Int.Cl.7) |
|---|---|
| Reason: | A61K47/48<br>A61K51/04<br>A61K49/00 |

Claims 1-6 encompass a genus of compounds defined only by their function wherein the relationship between the structural features of the members of the genus and said function have not been defined. In the absence of such a relationship either disclosed in the as-filed application or which would have been recognised based upon information readily available to one skilled in the art, the person skilled in the art would not know how to make and use compounds that lack structural definition. The fact that one could have assayed a compound of interest does not overcome this defect since one would have no knowledge beforehand as to whether or not any given compound (other than those that might be particularly disclosed in an application) would fall within the scope of what is claimed. Moreover, no assay has been described in the present application which would allow the skilled person to perform such selection. It would require undue experimentation (be an undue burden) to randomly screen undefined compounds for the claimed activity. Therefore, claims 1-6 do not fulfil the requirements of Art. 83 and Art. 84 EPC.

The applicant's attention is drawn to the fact that a search may be carried out during examination following a declaration of no search under Rule 45 EPC, should the problems which led to the declaration being issued be overcome (see EPC Guideline C-VI, 8.5).
-----

| Place of search | Date | Examiner |
|---|---|---|
| The Hague | 16 September 2004 | Dullaart, A |

EPO FORM 1504 (P04C37)